# EUROPEAN PATENT APPLICATION

(11) **EP 2 601 916 A1**
(43) Date of publication of application: **12.06.2013**
(21) Application number: 12196284.9
(22) Date of filing: 10.12.2012
(51) Int. Cl.: A61F 5/455

(54) **Sanitary system for women**

(30) Priority: 09.12.2011 NL 1039231
(71) Applicant: Botteram, Theo Remigius Maria, 7007 CC Doetinchem (NL)
(72) Inventor: Botteram, Theo Remigius Maria, 7007 CC Doetinchem (NL)
(74) Representative: Mink-Lindenburg, Charlotte Hildegard

(57) **Abstract**

The invention relates to a sanitary system for women for collecting and discharging urine, consisting of an anatomically shaped collecting member (1) to which discharge means are connectable. The anatomically shaped collecting member comprises a cup-like member (2) provided with a handle (3) and the discharge means are formed by a discharge hose (6) connected to the cup-like member as well as pump means and a receptacle connected to the pump means. According to the invention the cup-like member (2) has a form such that a woman can place the cup-like member (2) close to the pubic area while clothed in order to collect and discharge urine.

## Description

The invention relates to a sanitary system for women for collecting and discharging urine, provided with a collecting member comprising a cup-like member provided with a handle, wherein a discharge hose and pump means are connectable to the cup-like member.

A sanitary system for collecting and discharging urine is known from US 2,968,046. In the known system the discharge hose is connected to a sewage system, while the pump means comprise a water jet air pump connected to the mains water supply. The pump means are set into operation by opening a tap forming part of the water supply, after which the sanitary system can be utilized by the woman. In this form the sanitary system is highly suitable for use by for instance bedridden patients. It does however require structural modifications and is not displaceable.

The sanitary system according to the invention has for its object to achieve mobile use, for instance for the purpose of serving large groups of women, such as at festivals, where there are often long queues for the women's toilets and hygiene often leaves something to be desired.

A sanitary system according to the preamble of claim 1 is known from GB 2062472. The known sanitary system is intended for mobile use for bedridden patients, but is unsuitable for women.

The sanitary system according to the invention has the feature that the cup-like member has a form such that a woman can use the cup-like member to collect and discharge urine while clothed. The unique ergonomic shape enables highly practical use by women by means of placing the sanitary system close to the pubic area, in or under clothing.

It is noted for the sake of completeness that a sanitary system for women for collecting and discharging urine, provided with a collecting member comprising a cup-like member provided with a handle, is known from WO 9940879. The known sanitary system comprises an integrated pump and is connectable to a discharge hose. The known sanitary system is intended for (re)use by a private owner and takes a portable form in a foldable embodiment.

Also supplied is a spout-like disposable bag for collecting and storing urine for testing. The known sanitary system is neither intended nor suitable for placing in or under clothing during use. Nor is the known sanitary system suitable for bedridden female patients.

In a first preferred embodiment of the sanitary system according to the invention the cup-like member is provided with a peripheral edge extending inward from the sides of the cup-like member so as to define an opening. Owing to the overhanging peripheral edge the sanitary system can be held in diverse positions during use without the possibility of collected urine flowing out of it.

In a further preferred embodiment of the sanitary system according to the invention the cup-like member is provided with a discharge tube for connection to the discharge hose and pump means, which discharge tube debouches under the peripheral edge on the front side of the cup-like member. The sanitary system can now be used even in prone position without the possibility of collected urine flowing out of it.

A favourable embodiment of the inventive system has the feature that the handle is pivotally connected to the cup-like member. The collecting member can then be used without it being necessary here to remove clothing, wherein the cup-like member is pushed under the clothing and wherein the collecting member can follow the natural form of the body owing to the pivotable attachment to the handle. The advantage is that time is saved and that the user is obliged to proceed with care. Maximum privacy is moreover ensured in this manner.

A further favourable embodiment has the feature that the collecting member comprises an operating member arranged on or in the handle for activating the pump means when urine has to be discharged. The operating member can here comprise a switch or closing means which is arranged on or in the handle and which can be operated by the user.

A further favourable embodiment has the feature that a supply hose is connectable to the cup-like member and that an additional operating member is accommodated on or in the handle for supplying clean water to the cup-like member, so that the system can also be utilized as bidet.

A favourable alternative embodiment has the feature that the cup-like member comprises detection means for activating the pump means when urine has to be discharged, whereby the operating member becomes unnecessary.

A further favourable embodiment with which hygiene is guaranteed for a user has the feature that the system also comprises covering means for wholly or partially enclosing and covering the cup-like member. The covering means can for instance be made available via a dispenser and can be hooked onto the cup-like member with one movement of the hand or pushed over the cup-like member and subsequently adhered, clamped and/or fastened.

A further favourable embodiment has the feature that the covering means are manufactured at least partially from an absorbent material. Natural materials such as paper or cotton fibres are preferably used here, whereby the produced waste can be reused in simple manner.

A further favourable embodiment with which the comfort for the user can be increased and with which the chance of leakage is reduced has the feature that the covering means are each provided with an opening at least substantially corresponding to an opening in the cup-like member, and that the opening is provided with an edge manufactured from an absorbent material.

A further favourable embodiment has the feature that the edge is provided with a ribbed surface, whereby the wiping function is improved and whereby an underpressure can moreover never occur in the cup-like member, which underpressure could be perceived by the user as unpleasant.

The invention also relates to a collecting member as part of a system as specified in the foregoing paragraphs.

The invention also relates to a public sanitary facility, comprising a housing with one or more walk-in positions, wherein each walk-in position is provided with a sanitary system according to the invention.

The invention will now be further elucidated with reference to the following figures, wherein:
- Fig. 1A: is a schematic top view of a possible embodiment of a collecting member according to the invention;
- Fig. 1 B: shows a schematic cross-section of this collecting member;
- Fig. 2: shows schematically a possible embodiment of a system according to the invention;
- Fig. 3A: is a schematic top view of the collecting member provided with a covering means;
- Fig. 3B: shows a schematic cross-section of the collecting member provided with detection means;
- Fig. 3C: shows a schematic cross-section of the collecting member provided with an alternative covering means and with a spray nozzle;
- Fig. 4: is a schematic side view of a further possible embodiment of a system according to the invention.

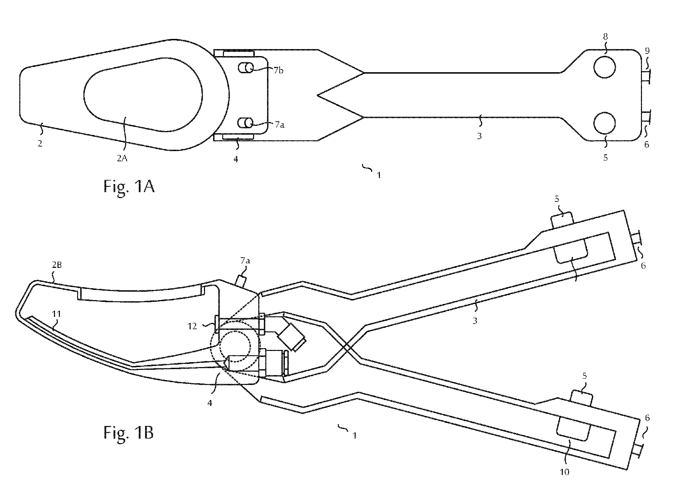

Fig. 1A is a schematic top view of a possible embodiment of a collecting member 1 according to the invention comprising a cup-like member 2 and a handle 3 connected to cup-like member 2 using a hinge connection 4. The object is to place cup-like member 2, optionally after loosening tightly fitting clothing, such that urine can be collected. A push-button 5 is then pressed which operates a switch or valve such that liquid is suctioned out of collecting member 2 using a pump (not shown in the figure). The suctioned liquid is discharged via a discharge hose 6. For reasons of hygiene cup-like member 2 is covered in a situation of use with a covering and wiping means (not shown in the figure) manufactured for instance from absorbent paper, which can be hooked beforehand onto two protrusions 7a, 7b and deposited in a waste bin after use. In the embodiment shown here collecting member 1 is provided with an additional push-button 8 which operates a switch or valve such that clean flushing water is supplied via a supply hose 9 and is sprayed into cup-like member 2, after which it is discharged via discharge hose 6. Collecting member 1 is manufactured here from light metal or plastic and, if desired, can be rinsed under a tap before or after use.

Fig. 1 B shows a schematic cross-section of this collecting member 1 with cup-like member 2 and handle 3 connected to cup-like member 2 using a hinge connection 4. Further shown is push-button 5, which here operates a valve 10 so that liquid can be suctioned out of collecting member 2 using a discharge tube 11 and discharged using discharge hose 6. Also shown is a spray nozzle 12 via which clean flushing water can be sprayed into collecting member 2 by pressing additional push-button 8. Also visible are protrusions 7a, 7b to which a covering and drying means can be hooked, wherein protrusions 7a, 7b also realize a centering function such that the covering and drying means cannot shift during use. It is of course also possible to replace protrusions 7a, 7b with a clamping member or to provide the covering and drying means with an adhesive edge.

Cup-like body 2 is anatomically shaped to the female body. Cup-like member 2 has a form such that a woman, while clothed, can place member 2 close to the pubic area in order to collect and discharge urine. Member 2 has a narrowed portion which can be placed inside underclothing. The dimensions of member 2 are chosen such that member 2 fits easily between the upper legs of a female body.

Member 2 is provided with a peripheral edge 2B extending inward from the sides of cup-like member 2. Edge 2B partially covers member 2 and defines a smaller opening 2A.

Cup-like member 2 is provided with a discharge tube 11 for connection to discharge hose 6 and pump means 13 and which debouches under peripheral edge 2B on the front side of cup-like member 2.

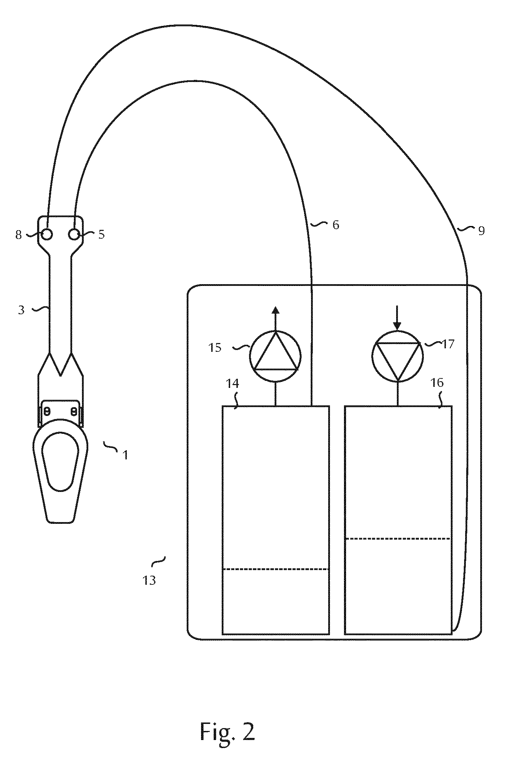

Fig. 2 shows schematically a possible embodiment of a system according to the invention consisting of a collecting member 1 and a pump unit 13 which are mutually connected by discharge hose 6 and supply hose 9. Placed in pump unit 13 are a dirty water tank 14, to which discharge hose 6 is connected, and a first pump 15 which maintains an underpressure of for instance 500 millibar in the dirty water tank 14 so that, when push-button 5 on handle 3 is pressed, liquid which may be present in cup-like member 2 will immediately be suctioned away via suction tube 11 and end up in the dirty water tank 14. Also placed in pump unit 13 are a clean water tank 16, to which supply hose 9 is connected, and a second pump 17 which maintains an overpressure of for instance 1500 millibar in the clean water tank 16 so that, when push-button 8 on handle 3 is pressed, clean water can be sprayed into cup-like member 2 via spray nozzle 12. In a stationary arrangement supply hose 9 can, if desired, be connected directly to the mains water supply.

Pump unit 13 can take many possible forms, depending on the location where the system is applied, for instance in the form of a carry case or portable unit, so that it can be attached to a bed or transported in a car. For use in for instance a train, the housing and tank 16 and pump 17 can generally be built in. For use in for instance a hospital, pump unit 13 can be provided with a handle and wheels.

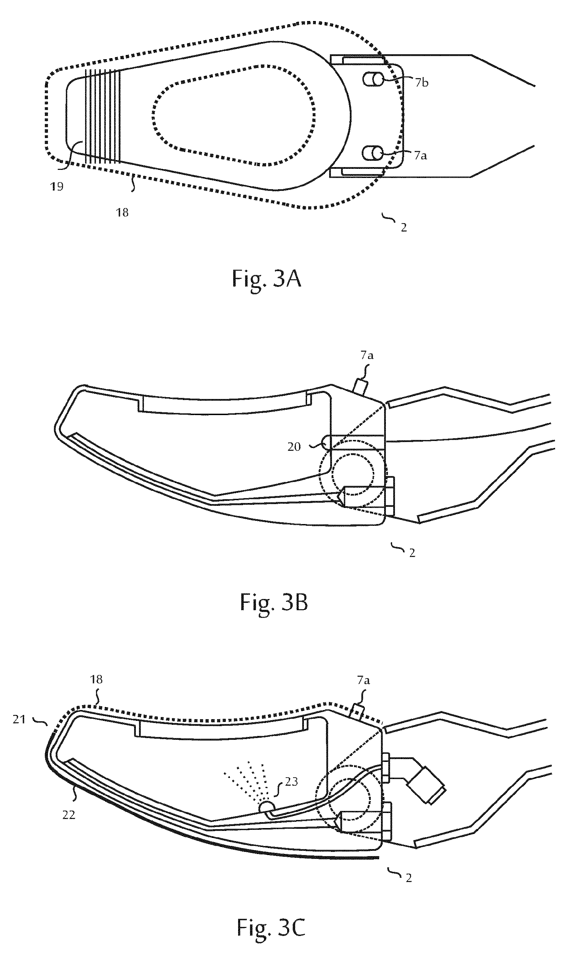

Fig. 3A is a schematic top view of cup-like member 2 provided with a covering means 18 shown with a broken line and consisting of an absorbent material such as paper, felt or an oval ring pressed from cotton wool with a thickness of 1-5 millimetres and which is provided with a central opening which in a position of use coincides with a central opening in cup-like member 2 so that on the one hand the edge of the cup-like member cannot be touched and on the other hand covering means 18 remains substantially dry during use. Covering means 18 is further provided with two fitting holes which drop round protrusions 7a, 7b in a position of use so that shifting of covering means 18 is impossible. Covering means 18 can then be arranged and removed without touching cup-like member 2. In the embodiment shown here covering means 18 is provided with a surface with ribs 19, whereby the wiping function is improved and with which an underpressure which feels unpleasant is moreover prevented from occurring in cup-like member 2. If desired, covering means 18 can be provided on the front side with an adhesive strip.

Fig. 3B shows a schematic cross-section of an embodiment wherein cup-like member 2 is provided with selection means, embodied here as a light-sensitive cell or a glass fibre 20, which is connected in further self-evident manner to a valve such that liquid is suctioned out of collecting member 2 as soon as the cup-like member is clearly in a situation of use in which no light can penetrate into cup-like member 2. The valve can be operated in wholly similar manner using a moisture sensor arranged in cup-like member 2.

Fig. 3C shows schematically an alternative covering means 21 consisting of a covering means 18 as according to Fig. 3A which is adhered onto a transparent plastic or paper bag 22 which is likewise provided with a central opening which coincides in a position of use with the central opening in cup-like member 2. Covering means 21 can then be arranged and removed substantially without touching cup-like member 2, and it is also impossible to touch cup-like member 2 during use. Collecting member 1 is further provided here with a spray nozzle 23. After pressing the additional push-button 8 clean flushing water supplied by supply hose 9 can be sprayed so that collecting member 1 can also be employed as handheld bidet. The flushing water is discharged here via discharge hose 6.

Other materials, including textile, are also suitable for the covering means as well as the stated materials. Textile has the advantage that it is washable and therefore very suitable for use by one person. The covering means can consist of one material or of a combination of several materials.

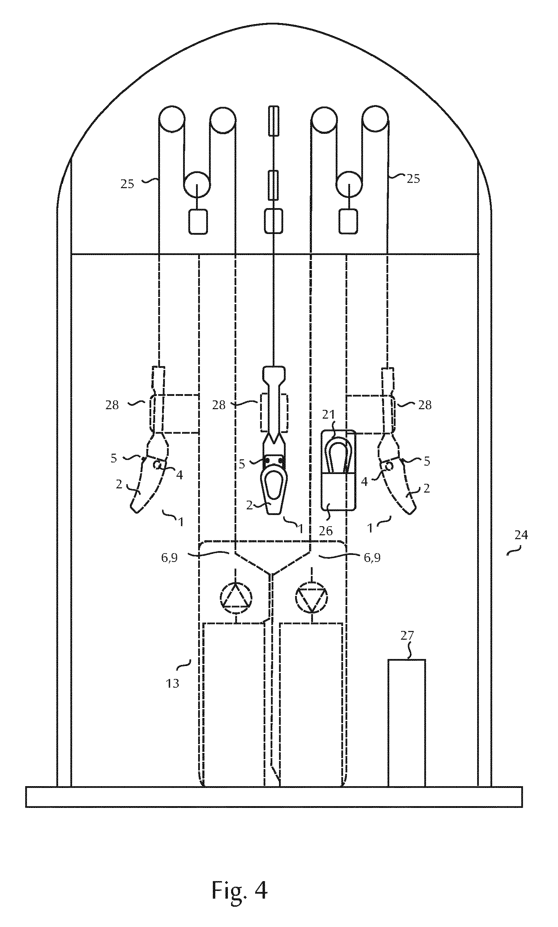

Fig. 4 is a schematic side view of a further possible application of a system according to the invention, consisting of a four-segment public urinal 24 for women having four standing areas separated by partitions and constructed around a central pump unit 13. Provided per segment is a collecting member 1 which is suspended from a combined conduit 6, 9 which is connected via a balancer 25 to central pump unit 13. A user removes a covering means 21 from a dispenser 26 and slides it over cup-like member 2. She then partially loosens her clothing if necessary and places cup-like member 2 in position, this being made possible by flexible connection 4. Push-button 5 is then pressed and urine can be discharged. Flushing is possible if desired by pressing button 8, wherein cup-like member 2 is flushed and suctioned empty. Collecting member 1 is then removed and covering means 21 is deposited in a container 27. Finally, collecting member 1 is snapped into a holder 28 at user height so that the following user can easily slide a covering means 21 therearound.

The number of segments or walk-in positions of the public urinal or the public sanitary facility 24 can of course be adapted to the conditions. In addition, the public sanitary facility 24 can be provided with a cleaning unit for cleaning each cup-like member 2 with water and soap or a disinfectant, both on the outside and on the inside. A drying unit can optionally be added to the cleaning unit.

## Claims

1. Sanitary system for women for collecting and discharging urine, provided with a collecting member (1) comprising a cup-like member (2) provided with a handle (3), wherein a discharge hose (6) and pump means (13) are connectable to the cup-like member, **characterized in that** the cup-like member (2) has a form such that a woman can place the cup-like member (2) close to the pubic area while clothed in order to collect and discharge urine.

2. Sanitary system as claimed in claim 1, wherein the cup-like member (2) is provided with a peripheral edge (2B) extending inward from the sides of the cup-like member (2) so as to define an opening (2A).

3. Sanitary system as claimed in claim 2, wherein the cup-like member (2) is provided with a discharge tube (11) for connection to the discharge hose (6) and pump means (13), which discharge tube (11) debouches under the peripheral edge (2B) on the front side of the cup-like member (2).

4. Sanitary system as claimed in any of the foregoing claims, **characterized in that** the handle (3) is pivotally connected to the cup-like member (2).

5. Sanitary system as claimed in any of the foregoing claims, **characterized in that** the collecting member (1) comprises an operating member (5) accommodated on or in the handle (3) for activating the pump means (13) when urine has to be discharged.

6. Sanitary system as claimed in any of the foregoing claims, **characterized in that** a supply hose (9) is connectable to the cup-like member and that an additional operating member (8) is accommodated on or in the handle (3) for supplying clean water to the cup-like member (2).

7. Sanitary system as claimed in any of the foregoing claims, **characterized in that** the cup-like member (2) comprises detection means (20) for activating the pump means (13) when urine has to be discharged.

8. Sanitary system as claimed in any of the foregoing claims, **characterized in that** the system also comprises covering means (18, 21) for wholly or partially enclosing and covering the cup-like member (2).

9. Sanitary system as claimed in claim 8, **characterized in that** the covering means (18, 21) are manufactured at least partially from an absorbent material.

10. Sanitary system as claimed in claim 9, **characterized in that** the covering means (18) are provided with an opening at least substantially corresponding to an opening (2A) in the cup-like member (2), and that the opening is provided with an edge manufactured from an absorbent material.

11. Collecting member (1) as described as part of the system as claimed in any of the foregoing claims.

12. Public sanitary facility, comprising a housing with one or more walk-in positions, wherein each walk-in position is provided with a sanitary system as claimed in any of the foregoing claims 1-10.
